(19) Europäisches Patentamt
European Patent Office
Office européen des brevets

(11) Veröffentlichungsnummer: **0 245 646 B1**

# (12) EUROPÄISCHE PATENTSCHRIFT

(45) Veröffentlichungstag der Patentschrift: **11.09.91**

(51) Int. Cl.$^5$: **C07D 231/38**, C07D 401/04, A01N 43/56, A01N 43/40

(21) Anmeldenummer: **87105058.9**

(22) Anmeldetag: **06.04.87**

Die Akte enthält technische Angaben, die nach dem Eingang der Anmeldung eingereicht wurden und die nicht in dieser Patentschrift enthalten sind.

## (54) Verfahren zur Herstellung von 1-Aryl-5-amino-pyrazolen.

(30) Priorität: **17.04.86 DE 3612939**

(43) Veröffentlichungstag der Anmeldung:
**19.11.87 Patentblatt 87/47**

(45) Bekanntmachung des Hinweises auf die Patenterteilung:
**11.09.91 Patentblatt 91/37**

(84) Benannte Vertragsstaaten:
**BE CH DE FR GB IT LI NL**

(56) Entgegenhaltungen:
**EP-A- 0 167 028**
**DE-A- 2 701 316**
**DE-B- 1 065 850**

(73) Patentinhaber: **BAYER AG**

**W-5090 Leverkusen 1 Bayerwerk(DE)**

(72) Erfinder: **Gallenkamp, Bernd, Dr.**
**Claudiusweg 5**
**W-5600 Wuppertal 1(DE)**
Erfinder: **Arold, Hermann, Dr.**
**Falkenberg 151**
**W-5600 Wuppertal 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 245 646 B1

## Beschreibung

Die Erfindung betrifft ein neues Verfahren zur Herstellung von bekannten 1-Aryl-5-amino-pyrazolen, die als Zwischenprodukte für die Synthese von Verbindungen mit herbizider und insektizider Wirksamkeit verwendet werden können.

Es ist bereits bekannt, daß man 1-Aryl-5-amino-pyrazole erhält, indem man Arylhydrazine mit 2,3-Dibrompropionitril oder α-Chloracrylnitril umsetzt (vgl. J. Prakt. Chem., 321,93 (1979); DE-OS 27 01 316 und DD-OS 126 303). Nachteilig an diesem Verfahren sind jedoch der hohe Preis und die schlechte Verfügbarkeit des als Reaktionskomponente benötigten 2,3-Dibrompropionitrils und α-Chloracrylnitrils. Außerdem wirkt sich für die Verfahrensdurchführung insbesondere der während des Reaktionsverlaufs anfallende hohe Salzgehalt im Reaktionsgemisch äußerst nachteilig aus.

Ferner ist bekannt, daß man 1-Aryl-5-amino-pyrazole erhält, indem man Arylhydrazine mit Cyanacetylen umsetzt (vgl, Takedo Kenkyusho Ho, 1971, 30, 475 [CA:76:85737 (1972)]. Nachteilig an diesem sind wiederum der hohe Preis und die Zugänglichkeit des als Reaktionskomponente notwendige Cyanacetylens.

Weiterhin ist bekannt, daß man 1-Aryl-5-amino-pyrazole durch Umsetzung von Arylhydrazinen mit β-Dimethylaminoacrylnitril erhält (vgl. Helv,. Chim Acta, 48, 1754 und DE-OS 21 41 700).

Wiederum sind der hohe Preis und die schlechte Verfügbarkeit des β-Dimethylaminoacrylnitrils von Nachteil.

Außerdem ist bekannt, daß man 1-Aryl-5-amino-pyrazole erhält, indem man Isoxazol im alkalischen Medium zu Cyanacetaldehyd umsetzt, anschließend mit Arylhydrazinen im sauren Medium zu den Arylhydrazonen des Cyanacetaldehyds kondensiert und schließlich im alkalischen Medium cyclisiert (vgl. Chem. Ber. 42, 59, (1909)). Der Nachteil dieses Verfahrens besteht in der schlechten Gesamtausbeute und den allgemeinen Nachteilen einer mehrstufigen Reaktionsführung.

Schließlich ist bekannt, daß man 1-Aryl-5-amino-pyrazole erhält, indem man Malondialdehyddioxim mit salpetriger Säure und Arylhydrazin umsetzt (vgl. Liebigs Ann. 739, 139 (1969) und DE-OS 19 13 845). Nachteilig an diesem Verfahren sind der hohe Preis und die schlechte Verfügbarkeit des als Reaktionskomponente benötigte Malondialdehyddioxims.

Es wurde nun gefunden, daß man bekannte 1-Aryl-5-amino-pyrazole der allgemeinen Formel (I)

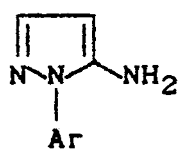

(I)

in welcher

Ar für gegebenenfalls substituiertes Phenyl oder Pyridyl steht,

erhält, wenn man Arylhydrazine der Formel (II)

$Ar\text{-}NH\text{-}NH_2$ (II)

in welcher

Ar die oben angegebene Bedeutung hat,

mit Acrylnitril der Formel (III)

$CH_2=CH\text{-}CN$ (III)

zunächst in einer ersten Stufe in Gegenwart eines Verdünnungsmittels und gegebenenfalls in Gegenwart eines Katalysators bei Temperaturen zwischen 20 °C und 100 °C umsetzt zu den Arylhydrazin-Derivaten der Formel (IV)

$Ar\text{-}NH\text{-}NH\text{-}CH_2\text{-}CH_2\text{-}CN$ (IV)

in welcher

Ar die oben angegebene Bedeutung hat,

und gegebenenfalls nach Zwischenisolierung in einer zweiten Stufe in Gegenwart von Luft oder Natriumhypochlorit als Oxidationsmittel und in Gegenwart einer Base bei Temperaturen zwischen 0 °C und 60 °C

oxidiert und cyclisiert.

Es ist als ausgesprochen überraschend zu bezeichnen, daß die Oxidation der Arylhydrazin-Derivate der Formel (IV) in Gegenwart eines so schwachen Oxidationsmittels wie Luft und die Cyclisierung in Gegenwart einer Base in so guten Ausbeuten gelingt, da aus dem Stand der Technik derartige Reaktionen nur in Gegenwart von starken Oxidationsmittel wie Eisensalzen bekannt sind (vgl. Helv. Chim. Acta, 41, 306 (1958)).

Das erfindungsgemäße Verfahren zeichnet sich durch eine Reihe von Vorteilen aus. So ermöglicht es die Herstellung von 1-Aryl-5-aminopyrazolen in hohen Ausbeuten und guter Reinheit, wobei die Ausgangsprodukte gut zugänglich sind. Ferner ist die Umsetzung äußerst einfach und wirtschaftlich in einem Eintropfverfahren durchführbar. Die Isolierung der 1-Aryl-5-amino-pyrazole bereitet keinerlei Schwierigkeiten, wohingegen bei der Oxidation durch Eisensalze die Aufarbeitung des Reaktionsgemisches durch die schwer abtrennbaren Niederschläge äußerst erschwert wird.

Mit Hilfe des erfindungsgemäßen Verfahrens erhält man vorzugsweise solche 1-Aryl-5-amino-pyrazole der Formel (I),

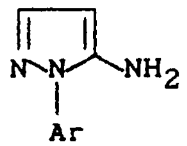 (I)

bei welchen

Ar für einfach oder mehrfach, gleich oder verschieden substituiertes Phenyl, für jeweils gegebenenfalls einfach oder mehrfach gleich oder verschieden substituiertes 2-Pyridyl, 3-Pyridyl oder 4-Pyridyl steht, wobei als Substituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen im Alkylteil, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^1$,
wobei

$R^1$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

p für eine Zahl 0, 1 oder 2 steht.

Das erfindungsgemäße Verfahren betrifft besonders bevorzugt Verbindungen der Formel (I),
in welcher

Ar für ein- bis fünffach, gleich oder verschieden substituiertes Phenyl oder für jeweils gegebenenfalls ein- bis vierfach gleich oder verschieden substituiertes 2-Pyridyl oder 4-Pyridyl steht, wobei als Phenyl- bzw. Pyridylsubstituenten jeweils in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_p-R^1$,
wobei

$R^1$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht und

p für eine Zahl 0, 1 oder 2 steht.

Verwendet man beispielsweise 2,6-Dichlor-4-trifluormethylphenylhydrazin und Acrylnitril als Ausgangsstoffe und Sauerstoff als Oxidationsmittel und Natriumhydroxid als Base, so kann der Verlauf des erfdindungsgemäßen Verfahrens durch das folgende Formelschema veranschaulicht werden:

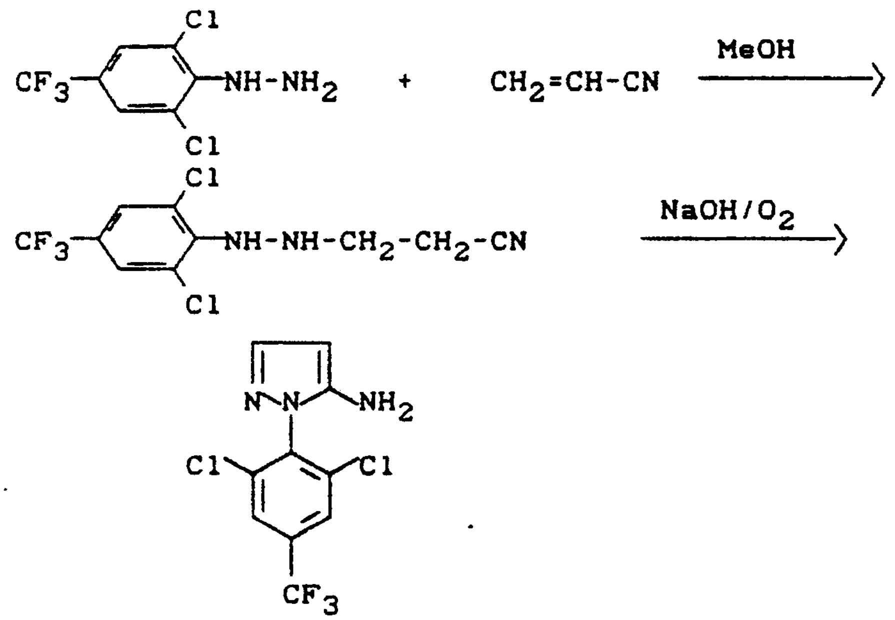


Die zur Durchführung des erfindungsgemäßen Verfahrens als Ausgangsstoffe benötigten Arylhydrazine sind durch die Formel (II) allgemein definiert. In dieser Formel (II) steht Ar vorzugsweise für diejenigen Reste, die bereits im Zusammenhang mit der Beschriebung der Endprodukte der Formel (I) als bevorzugt für diesen Substituenten genannt wurden.

Die Arylhydrazine der Formel (II) sind bekannt (vgl. z.B. US-PS 4 127 575; US-PS 3 609 158; DE-OS 2 558 399; J. Chem. Soc. C 1971, 167) bzw. können nach bekannten Verfahren in einfacher, analoger Weise hergestellt werden (vgl.: z.B. Houben-Weyl "Methoden der organischen Chemie" Band X/2,S.203, Thieme Verlag Stuttgart 1967), indem man beispielsweise die entsprechenden Amine mit Natriumnitrit, in Gegenwart einer Säure, wie beispielsweise Schwefelsäure, und dann mit Zinn-(II)-chlorid, ebenfalls in Gegenwart einer Säure, wie beispielsweise Salzsäure, bei Temperaturen zwischen -20 °C und +80 °C umsetzt.

Das Acrylnitril der Formel (III) ist eine allgemein bekannte Verbindung der organischen Chemie.

Das erfindungsgemäße Verfahren wird in Gegenwart eines Verdünnungsmittel durchgeführt. Hierzu gehören insbesondere aliphatische oder aromatische, gegebenenfalls halogenierte Kohlenwasserstoff, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid. Besonders bevorzugt verwendet man Ethanol oder Methanol als Lösungsmittel.

Die Reaktionstemperaturen können bei der Dürchführung des erfindungsgemäßen Verfahrens in einem größeren Bereich variiert werden. Im allgemeinen arbeitet man bei der ersten Stufe bei Temperaturen zwischen 20°C und 100°C, vorzugsweise bei Temperaturen zwischen 40°C und 80°C. Bei der zweiten Stufe arbeitet man im allgemeinen bei Temperaturen zwischen 0°C und 60°C, vorzugsweise bei Temperaturen zwischen 10°C und 50°C.

Die 1. Stufe des erfindungsgemäßen Verfahrens kann gegebenenfalls in Gegenwart eines Katalysators durchgeführt werden, vorzugsweise arbeitet man in Gegenwart eines Katalysators. Hierzu gehören vorzugsweise das Dinatriumsalz der Ethylendiamintetraessigsäure (Titriplex III), Alanin und Benzyltrimethylammoniumhydroxid (Triton B).

Die 2. Stufe des erfindungsgemäßen Verfahrens erfordert die Gegenwart von Natriumhypochlorit oder Luft als Oxidationsmittel.

Die 2. Stufe des erfindungsgemäßen Verfahrens wird in Gegenwart einer Base durchgeführt. Hierzu gehören vorzugsweise Alkalimetallhydroxide wie z.B. Natrium- und Kaliumhydroxid sowie Alkalimetallalkoholate wie z.B. Natrium-und Kaliummethylat.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man in der 1. Stufe pro Mol Arylhydrazin

der Formel (II) im allgemeinen 1 bis 5 Mol, vorzugsweise 1 bis 3 Mol Acrylnitril sowie in der 2. Stufe im allgemeinen 1 bis 4 Mol, vorzugsweise 1 bis 3 Mol Oxidationsmittel und im allgemeinen 0,1 bis 1,0 Mol, vorzugsweise 0,4 bis 0,5 Mol Base ein.

Die Reaktion wird so durchgeführt, daß man die Reaktionspartner in dem entsprechenden Verdünnungsmittel und gegebenenfalls in Gegenwart eines Katalysators für 24 bis 48 Stunden erhitzt und anschließend gegebenenfalls nach Zwischenisolation in Gegenwart des entsprechenden Oxidationsmittels, der entsprechenden Base und dem entsprechenden Verdünnungsmittel bei Temperaturen zwischen 20 °C und 40 °C für 6 bis 12 Stunden umsetzt.

Die Isolierung der 1-Aryl-5-amino-pyrazole der Formel (I) erfolgt in üblicher Art und Weise, indem man beispielsweise das Reaktionsgemisch neutral stellt, einengt, den Rückstand mit einem wasserunlöslichen organischen Lösungsmittel extrahiert, mit Wasser wäscht, trocknet und das organische Lösungsmittel destillativ entfernt.

Die nach dem erfindungsgemäßen Verfahren herstellbaren 1-Aryl-5-amino-pyrazole der Formel (I) sind bekannte Ausgangsstoffe zur Synthese von biologisch aktiven Verbindungen, wie z.B. zur Synthese von substituierten 5-Amino-1-phenyl-pyrazolen (vgl. DE-OS 34 02 308), welche gute herbizide Eigenschaften besitzen. In entsprechenden Aufwandmengen sind auch die nach dem erfindungsgemäßen Verfahren herstellbaren 1-Aryl-5-amino-pyrazole selbst herbizid wirksam (vgl. DE-OS 34 02 308).

So läßt sich beispielsweise das 5-Propionamid-1-(2,6-dichlor-4-trifluormethylphenyl)-pyrazol der Formel

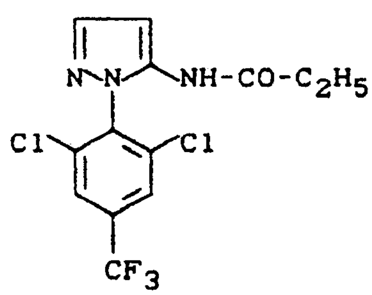

herstellen, indem man 5-Amino-1-(2,6-dichlor-4-trifluormethylpenyl)-pyrazol mit Propionylchlorid in Gegenwart von Dichlormethan und Pyridin umsetzt. Diese Synthese läßt sich formelmäßig wie folgt veranschaulichen:

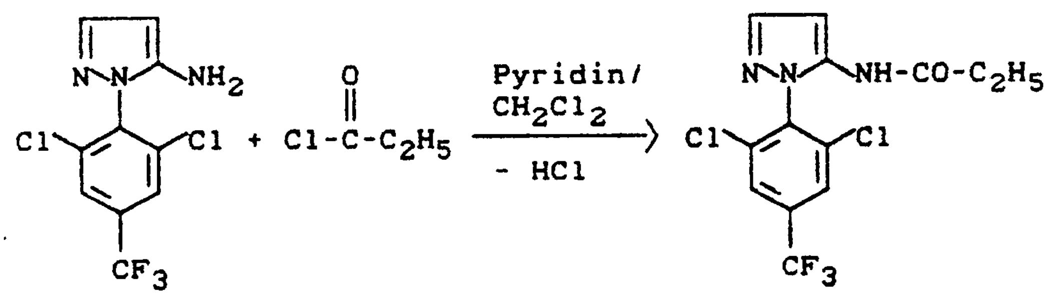


Das erfindungsgemäße Verfahren wird durch die folgenden Beispiele veranschaulicht.

Beispiel 1

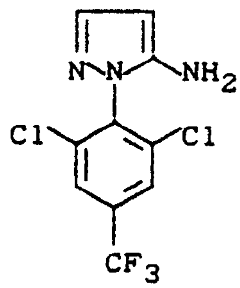

(Stufe 1 und 2 als Eintopfverfahren)

245 g (1 Mol) 2,6-Dichlor-4-trifluormethylphenylhydrazin, 60 g (1,14 Mol) Acrylnitril und 1 g Dinatriumsalz der Ethylendiamintetraessigsäure (Titriplex III) werden in 350 ml Methanol 24 Stunden unter Rückfluß erhitzt. Anschließend gibt man 20 g Natriumhydroxid zu und leitet bei 20 °C für 10 Stunden Luft durch die Reaktionsmischung. Danach wird das Reaktionsgemisch mit konzentrierter Salzsäure auf pH 7 gestellt, eingeengt, in 250 ml Toluol aufgenommen und zweimal mit je 500 ml Wasser gewaschen. Die organische Phase wird eingeengt und destilliert.

Man erhält 281 g (95 % der Theorie) an 1-(2,6-Dichlor-4-trifluormethylphenyl)-5-amino-pyrazol vom Schmelzpunkt 90 - 94 °C.

Herstellung der Ausgangsverbindung:

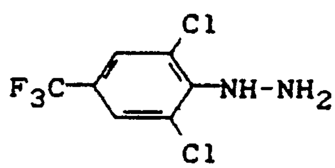

6,2 g (0,025 Mol) 3,4,5-Trichlor-trifluormethylbenzol und 6,25 g (0,125 Mol) Hydrazinhydrat werden in 12 ml Pyridin 48 Stunden bei 115 - 120 °C unter Rückfluß erhitzt. Zur Aufarbeitung destilliert man das Lösungsmittel ab, nimmt den Rückstand in Wasser auf und extrahiert dreimal mit jeweils ca. 30 ml Dichlormethan. Die vereinigten organischen Phasen werden über Magnesiumsulfat getrocknet, im Vakuum eingeengt und anschließend destilliert.

Man erhält 5,1 g (83 % der Theorie) an 2,6-Dichlor-4-tri-fluormethylphenylhydrazin vom Schmelzpunkt 56 bis 57 °C mit einem gaschromatographisch bestimmten Gehalt von 90 %.

Beispiel 2

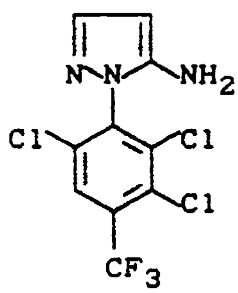

Stufe 2

Zu 13,5 (0,04 Mol) N-(2,3,6-Trichlor-4-trifluormethyl-phenyl)-N'-2-cyanethylhydrazin in 80 ml Ethanol läßt man bei 10 °C 38 g Chlorlauge (hergestellt durch Einleiten von ca. 18 g Chlor in 220 g 20 %iger

Natriumlauge) zutropfen.

Man rührt 16 Stunden bei 20 °C, gibt 0,5 g festes Natriumhydroxid zu und rührt 6 Stunden nach. Man engt ein, nimmt in Methylenchlorid auf, wäscht zweimal mit Wasser, engt ein und destilliert. Man erhält 10 g (76 % der Theorie) 1-(2,3,6-Trichlor-4-trifluormethylphenyl)-5-amino-pyrazol.

$^1$H-NMR ($CDCl_3$) : $\delta$ = 7,85 (1H); 7,55, 5,7 (2H); 3,6 (2H)

Herstellungsbeispiel für eine herbizid wirksame Verbindung

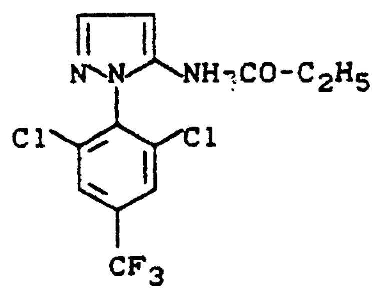

14,8 g (0,05 Mol) 5-Amino-1-(2,6-dichlor-4-trifluormethylphenyl)-pyrazol in 100 ml Dichlormethan werden bei Raumtemperatur unter Rühren nacheinander mit 5 ml (5,3 g / 0,05 Mol) 98%igem Propionylchlorid und dann mit 5 ml (5,0g / 0,063 Mol) wasserfreiem Pyridin versetzt. Dabei steigt die Temperatur auf 40 °C. Nach beendeter Zugabe rührt man weitere 16 Stunden bei Raumtemperatur, gibt 50 ml Dichlormethan zu, wäscht je zweimal mit 100 ml Wasser, 100 ml gesättigter Natriumhydrogencarbonatlösung und 100 ml Natriumchloridlösung, trocknet über Magnesiumsulfat und entfernt das Lösungsmittel im Vakuum. Der feste Rückstand wird mit wenig Hexan gewaschen und getrocknet.

Man erhält 12,2 g (69,3 % der Theorie) an 5-Propionamido1-(2,6-dichlor-4-trifluormethylphenyl)-pyrazol vom Schmelzpunkt 125 °C.

## Patentansprüche

1. Verfahren zur Herstellung von 1-Aryl-5-amino-pyrazolen der Formel (I)

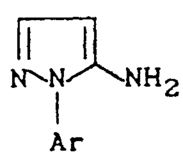 (I)

in welcher

Ar für gegebenenfalls substituiertes Phenyl oder Pyridyl steht,

dadurch gekennzeichnet, daß man Arylhydrazine der Formel (II)

Ar-NH-$NH_2$ (II)

in welcher

Ar die oben angegebene Bedeutung hat,

mit Acrylnitril der Formel (III)

$CH_2$=CH-CN (III)

in Gegenwart eines Verdünnungsmittels umsetzt zu den Arylhydrazin-Derivaten der Formel (IV) (1. Stufe)

$Ar\text{-}NH\text{-}NH\text{-}CH_2\text{-}CH_2\text{-}CN$ (IV)

in welcher
Ar die oben angegebene Bedeutung hat,

und diese Verbindungen mit Luft oder Natriumhypochlorit als Oxidationsmittel oxidiert und in Gegenwart einer Base cyclisiert (2.Stufe).

**2.** Verfahren gemäß Anspruch 1, dadurch gekennzeichnet, daß man die erste Stufe in Gegenwart eines Katalysators durchführt.

**3.** Verfahren gemäß Anspruch 3, dadurch gekennzeichnet, daß man als Katalysator das Dinatriumsalz der Ethylendiamintetraessigsäure (Titriplex III, Alanin oder Benzyltrimethylammoniumhydroxid (Triton B) einsetzt.

**4.** Verfahren gemäß Anspruch 1, dadurch gekennzeichnet, daß man als Base Alkalimetallhydroxide oder Alkalimetallalkoholate verwendet.

**5.** Verfahren gemäß Anspruch 1, dadurch gekennzeichnet, daß man die erste Stufe bei Temperaturen zwischen 40 °C und 80 °C durchführt.

**6.** Verfahren gemäß Anspruch 1, dadurch gekennzeichnet, daß man die zweite Stufe bei Temperaturen zwischen 10 °C und 50 °C durchführt.

**7.** Verfahren gemäß Anspruch 1, dadurch gekennzeichnet, daß man in der 1. Stufe pro Mol Arylhydrazin der Formel (II) 1 bis 5 Mol Acrylnitril sowie in der 2. Stufe 1 bis 4 Mol Oxidationsmittel und 0,1 bis 1,0 Mol Base einsetzt.

**8.** Verfahren gemäß Anspruch 1, dadurch gekennzeichnet, daß man die Umsetzung als Eintopfverfahren durchführt.

**9.** Verfahren gemäß Anspruch 1, dadurch gekennzeichnet, daß man die Arylhydrazin-Derivate der Formel (IV) isoliert.

**10.** Verfahren gemäß Anspruch 1, dadurch gekennzeichnet, daß man 1-Aryl-5-amino-pyrazole der Formel (I) erhält

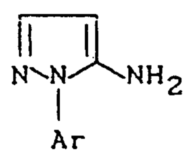

(I)

in welcher

Ar für einfach oder mehrfach, gleich oder verschieden substituiertes Phenyl, für jeweils gegebenenfalls einfach oder mehrfach gleich oder verschieden substituiertes 2-Pyridyl, 3-Pyridyl oder 4-Pyridyl steht, wobei als Substituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen im Alkylteil, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^1$,

wobei

$R^1$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

p für eine Zahl 0, 1 oder 2 steht.

**Claims**

**1.** Process for the preparation of 1-aryl-5-amino-pyrazoles of the formula (I)

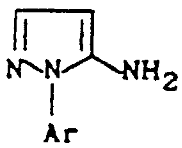

(I)

in which

Ar represents optionally substituted phenyl or pyridyl,

characterised in that arylhydrazines of the formula (II)

$Ar-NH-NH_2$ (II)

in which

Ar has the abovementioned meaning,

are initially reacted with acrylonitrile of the formula (III)

$CH_2=CH-CN$ (III)

in the presence of a diluent to give the arylhydrazine derivatives of the formula (IV) (1st stage)

$Ar-NH-NH-CH_2-CH_2-CN$ (IV)

in which

Ar has the abovementioned meaning,

and these compounds are oxidized using air or sodium hypochlorite as the oxidising agent and cyclised in the presence of a base (2nd stage).

**2.** Process according to Claim 1, characterised in that the first stage is carried out in the presence of a catalyst.

**3.** Process according to Claim 2, characterised in that the disodium salt of ethylenediaminetetraacetic acid (Titriplex III), alanine or benzyltrimethylammonium hydroxide (Triton B) is employed as the catalyst.

**4.** Process according to Claim 1, characterised in that alkali metal hydroxides or alkali metal alcoholates are used as the base.

**5.** Process according to Claim 1, characterised in that the first stage is carried out at temperatures between 40 °C and 80 °C.

**6.** Process according to Claim 1, characterised in that the second stage is carried out at temperatures

between 10° C and 50° C.

**7.** Process according to Claim 1, characterised in that 1 to 5 mol of acrylonitrile are employed in the 1st stage, per mol of arylhydrazine of the formula (II), and 1 to 4 mol of oxidising agent and 0.1 to 1.0 mol of base are employed in the 2nd stage.

**8.** Process according to Claim 1, characterised in that the reaction is carried out as a one-pot process.

**9.** Process according to Claim 1, characterised in that the arylhydrazine derivatives of the formula (IV) are isolated.

**10.** Process according to Claim 1, characterised in that 1-aryl-5-amino-pyrazoles of the formula (I)

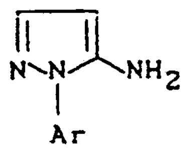

(I)

in which

Ar represents phenyl which is monosubstituted or polysubstituted by identical or different substituents, or represents 2-pyridyl, 3-pyridyl or 4-pyridyl, in each case optionally monosubstituted or polysubstituted by identical or different substituents, possible substituents in each case being: cyano, nitro, halogen, in each case straight-chain or branched alkyl, alkoxy and alkoxycarbonyl with in each case 1 to 4 carbon atoms in the alkyl moiety and also in each case straight-chain or branched halogenalkyl and halogenoalkoxy with in each case 1 to 4 carbon atoms and 1 to 9 identical or different halogen atoms, and a radical $-S(O)_p-R^1$

wherein

$R^1$ represents amino, or represents in each case straight-chain or branched alkyl, alkylamino, dialkylamino or halogenoalkyl with in each case 1 to 4 carbon atoms in the individual alkyl moieties and, in the case of the halogenoalkyl, with 1 to 9 identical or different halogen atoms

and

p represents the number 0, 1 or 2, are obtained.

## Revendications

**1.** Procédé pour la fabrication de 1-aryl-5-aminopyrazoles de formule (I) :

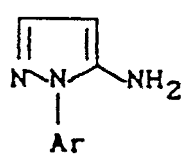

(I)

dans laquelle
Ar représente un groupe phényle ou pyridyle éventuellement substitué,
caractérisé en ce que l'on fait réagir des arylhydrazines de formule (II) :

$Ar-NH-NH_2$ (II)

dans laquelle
Ar a la signification indiquée ci-dessus, avec l'acrylonitrile de formule (II) :

$CH_2=CH\text{-}CN$ (III)

en présence d'un agent diluant pour donner les dérivés d'arylhydrazines de formule (IV) (le étape) :

$Ar\text{-}NH\text{-}NH\text{-}CH_2\text{-}CH_2\text{-}CN$ (IV)

dans laquelle
Ar a la signification indiquée ci-dessus,
et on oxyde ces composés par l'air ou par l'hypochlorite de sodium comme agent oxydant et on les cyclise en présence d'une base (2e étape).

2. Procédé selon la revendication 1, caractérisé en ce que l'on met en oeuvre ta première étape en présence d'un catalyseur.

3. Procédé selon la revendication 2, caractérisé en ce que l'on utilise comme catalyseur le sel disodique de l'acide éthylènediaminetétraacétique (Titriplex III), l'alanine ou l'hydroxyde de benzyltriméthylammonium (Triton B).

4. Procédé selon la revendication 1, caractérisé en ce que l'on utilise comme base des hydroxydes de métaux alcalins ou des alcoolates de métaux alcalins.

5. Procédé selon la revendication 1, caractérisé en ce que l'on met en oeuvre la première étape à des températures comprises entre 40 et 80° C.

6. Procédé selon la revendication 1, caractérisé en ce que l'on met en oeuvre la deuxième étape à des températures comprises entre 10 et 50° C.

7. Procédé selon la revendication 1, caractérisé en ce que l'on utilise dans la première étape par mole d'hydrazine de formule (II) 1 à 5 mol d'acrylonitrile et dans la deuxième étape 1 à 4 mol d'agent oxydant et 0,1 à 1,0 mol de base.

8. Procédé selon la revendication 1, caractérisé en ce que l'on met en oeuvre la réaction comme procédé en un pot.

9. Procédé selon la revendication 1, caractérisé en ce que l'on isole les dérivés d'arylhydrazines de formule (IV).

10. Procédé selon la revendication 1, caractérisé en ce que l'on obtient des 1-aryl-5-aminopyrazoles de formule (I) :

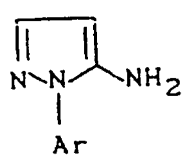

(I)

dans laquelle
Ar représente un groupe phényle substitué par un ou plusieurs substituants identiques ou différents, un groupe 2-pyridyle, 3-pyridyle ou 4-pyridyle chaque fois éventuellement substitué par un ou plusieurs substituants identiques ou différents, les substituants entrant en ligne de compte étant chaque fois : cyano, nitro, halogène, alkyle, alcoxy ou alcoxycarbonyle chaque fois à chaîne droite ou ramifiée, ayant chaque fois 1 à 4 atomes de carbone dans la partie alkyle, en outre halogénoalkyle ou halogénoalcoxy chaque fois à chaîne droite ou ramifiée, ayant chaque fois 1 à 4 atomes de carbone et 1 à 9 atomes

d'halogènes identiques ou différents ou un reste $-S(O)_p-R^1$,
ou
$R^1$ représente un groupe amino ou un groupe alkyle, alkylamino, dialkylamino ou halogénoalkyle à chaîne droite ou ramifiée ayant chaque fois 1 à 4 atomes de carbone dans les parties alkyles séparées et, dans le cas du reste halogénoalkyle, ayant chaque fois 1 à 9 atomes d'halogènes identiques ou différents et
p représente un nombre 0, 1 ou 2.